# EUROPÄISCHE PATENTSCHRIFT

(11) **EP 0 828 455 B1**
(45) Veröffentlichungstag und Bekanntmachung des Hinweises auf die Patenterteilung: **22.08.2001**
(21) Anmeldenummer: 96913424.6
(22) Anmeldetag: 21.05.1996
(51) Int. Cl.: A61B 17/15

(54) **INSTRUMENTARIUM FÜR UMSTELLUNGS-OSTEOTOMIE DER UNTEREN EXTREMITÄT**
INSTRUMENTS FOR ADJUSTMENT OSTEOTOMY OF THE LOWER EXTREMITY
ENSEMBLE D'INSTRUMENTS POUR OSTEOTOMIE CORRECTIVE DE L'EXTREMITE INFERIEURE

(30) Priorität: 26.05.1995 CH 155695
(43) Veröffentlichungstag der Anmeldung: 18.03.1998
(73) Patentinhaber: Mathys Medizinaltechnik AG, 2544 Bettlach (CH)
(72) Erfinder: WEHRLI, Ulrich, CH-3084 Wabern (CH)
(74) Vertreter: Lusuardi, Werther Giovanni, Dr.
(86) Internationale Anmeldenummer: CH9600195
(87) Internationale Veröffentlichungsnummer: WO9637154

(56) Entgegenhaltungen:
- EP-A- 0 231 885
- FR-A- 2 679 126
- US-A- 4 349 018
- US-A- 4 627 425

## Beschreibung

Die Erfindung betrifft ein Instrumentarium zur Unterstützung einer orthopädisch-chirurgischen Operation zur Korrektur der mechanischen Belastungsverhältnisse im Knie gemäss Oberbegriff des Patentanspruches 1.

Die gezielte Korrektur der mechanischen Belastungsverhältnisse bei einseitiger degenerativer Erkrankung des Knies ist eine bekannte und vielfach verwendete Methode um Schmerzfreiheit und Beweglichkeit ohne prothetische Versorgung wiederherzustellen. Durch einen orthopädischen-chirurgischen Eingriff wird dabei die Achsenstellung der unteren Extremität so verändert, dass eine Entlastung erkrankter Gelenksegmente erreicht wird. Die am häufigsten verwendete Variante dieser Operation ist die lateral angesetzte, valgisierende Tibia-Kopf-Osteotomie bei vorhandener Schädigung des medialen Gelenkanteils. Dabei wird durch Entnahme eines keilförmigen Knochenspanes eine Veränderung der Beinachse hin zur X-Beinstellung erzeugt und so das erkrankte, mediale Gelenk-Kompartiment entlastet. Voraussetzung für den klinischen Erfolg der Umstellungs-Osteotomie ist neben der korrekten Indikationsstellung die exakte Bestimmung des Korrekturwinkels, die Entnahme eines zum Korrekturwinkel korrespondierenden, keilförmigen Knochenspanes, die Qualität der Osteotomieflächen und die stabile mikrobewegungsfreie Osteosynthese mittels eines geeigneten Osteosynthese-Implantates.

Im Falle der medialen Gonarthrose wird durch die Umstellung die mechanische Achse, die beim gesunden Knie eine Gerade durch die Gelenkzentren von Hüft-, Knie- und Sprunggelenk bildet, um 20 bis 30 mm hin zum lateralen Gelenkanteil verlegt. Der Korrekturwinkel summiert sich aus der vorhandenen Fehlstellung und der Überkorrektur der mechanischen Achse. Durch diese Überkorrektur wird die erkrankte Gelenkhälfte entlastet und die gegenüberliegende, gesunde Hälfte etwas stärker belastet. Der zu entnehmende Knochenkeil kann entweder horizontal, d.h. senkrecht zur Diaphysenachse oder in einem Winkel dazu entnommen werden. Bei dem erwähnten Instrumentarium wählen wir einen schräg zur erkrankten Seite hin ansteigenden Osteotomie-Verlauf, um den Drehpunkt der Osteotomie in den dichten subchondralen Knochen, wenige Millimeter von der seitlich begrenzenden Kortikalis festzulegen. Die so erhaltene Knochenbrücke ergibt einen Zuggurtungseffekt und erlaubt die einfache Osteosynthese der Osteotomie mittels gegenseitig angebrachtem Implantat. Zur Erzeugung dieser für die Stabilität wichtigen Knochenbrücke ist die gezielte Wahl des generellen Osteotomie-Winkels relativ zur Gelenkachse wichtig, da der Kreuzungspunkt der keilförmigen Osteotomie im dichten Knochenvolumen unmittelbar unterhalb des Gelenkes gute Voraussetzungen für einen ungestörten Heilungsverlauf, ohne sekundären Korrekturverlust gewährt.

Zur Planung des Eingriffs wird eine lange Röntgenaufnahme der gesamten Extremität, auf das Gelenk zentrierte Aufnahme verwendet, wobei zur Vermeidung von Projektionsfehlern die Aufnahmeebene möglichst exakt der Frontalebene zu entsprechen hat. Bei der Vermessung der Röntgenbilder ist der Vergrösserungsfaktor zu berücksichtigen. Da Röntgenbilder von der Strahlmitte ausgehend mit einem nichtlinearen Vergrösserungsfaktor behaftet sind und die Aufnahmeebene durch Positionier-Ungenauigkeit in der Regel nicht exakt in der Ebene der Körperachsen liegt ist diese Planung immer fehlerbehaftet. Gerade bei Patienten fortgeschrittenen Alters ist die Aufnahmetechnik oft besonders schwierig. Besonders bei gegebener Flexionskontraktur wirken sich bereits geringe Rotationsabweichungen von der Frontal-Ebene derart aus, dass die zur Erzielung eines optimalen Ergebnisses erforderlichen Genauigkeit bereits in der Planung nicht mehr gegeben ist. Zur Umsetzung des Korrekturwinkels aus der Planung und zur Führung des Sägewerkzeuges sind winkelgebende Instrumente, die in der Regel am Skelett befestigt werden und über Einrichtungen zur Führung eines maschinell angetriebenen Sägeblattes verfügen gebräuchlich.

Die Autoren der europäischen Patentanmeldung EP 0 231 885 A1 schlagen die Befestigung eines kreisbogenförmigen, in Winkelgraden skalierten Instrumentes mittels zweier oberhalb und unterhalb der Osteotomie in der Tibia zu positionierenden Stifte vor. Die Stifte sind gegeneinander im Keilwinkel der Osteotomie angestellt. Diese Stifte tragen je eine ebene Platte, die gegen den Knochen positioniert, die Führung der Säge bilden. Damit sind Winkel und Ebene der keilförmig zusammenlaufenden Osteotomie-Flächen festgelegt. Nicht festgelegt ist der Ort des Zusammentreffens der Sägeschnitte in der Spitze des zu entfernenden Knochenkeils. Dieser soll idealerweise einige Millimeter vor der gegenüberliegenden kortikalen Schale in der dichten gelenknahen Spongiosa der Tibia liegen, damit zum einen nach der Keilentnahme die Osteotomieflächen über den gesamten Knochenquerschnitt schliessbar sind, zum anderen ein minimales Knochenvolumen, verbunden mit einer möglichst geringen Verkürzung der Beinlänge, entnommen wird. Der Erhalt einer dünnen, deformierbaren Knochenbrücke ist wünschenswert und gewährleistet die sichere Zuordnung der Osteotomie-Segmente und erhöht die Stabilität der folgenden Osteosynthese im Sinne einer Zuggurtung. Beim in EP 0 231 885 A1 vorgeschlagenen Instrument ist dieser Kreuzungspunkt im Röntgenbild zu ermitteln und das Instrument muss in der Operation nach dieser Planung in Distanz und Orientierung an der Tibia angebracht werden. Ort der Kreuzung der Schnittflächen ist bei dieser Ausführung der Mittelpunkt des winkelgebenden Kreisbogensegmentes des Instrumentes.

In der französischen Patentanmeldung FR 2,679,126 ist eine ähnliche Anordnung beschrieben, die ebenfalls mittels Stiften am Skelett befestigt wird und Osteotomien ermöglicht, die in Folge über eine exakt auf einer kreissegmentförmigen Schiene geführte und in Winkelgraden schwenkbare Sägeführung die Entnahme des Knochenkeils mittels oszillierender Knochensäge ermöglicht. Auch bei dieser Vorrichtung muss der Kreuzungspunkt der Sägeschnitte auf dem Röntgenbild geplant und auf die Situation in der Operation übertragen werden.

Wie vorausgehend beschrieben, beinhaltet die Planung anhand des Röntgenbildes bereits einige unvermeidbare Ungenauigkeiten. Zusätzlich ist zu erwarten, dass bei der Positionierung eines der oben beschriebenen Instrumente am Patienten sich weitere Ungenauigkeiten bezüglich der Lage des Osteotomie-Keils ergeben, insbesondere da das Ergebnis der Positionierung nicht direkt überprüfbar ist.

Aufgabe der Erfindung ist es, ein Instrumentarium zu schaffen, welches erlaubt korrigierende, keilförmige Osteotomien an der unteren Extremität mit hoher Genauigkeit auszuführen, wobei das Resultat unabhängig ist von Ungenauigkeiten, die mit der Planung mittels Röntgenbildern verbunden sind. Zusätzlich soll der Verlauf der Knochenschnitte, insbesondere deren Kreuzungspunkt exakt festlegbar und vor deren Ausführung überprüfbar sein.

Diese Aufgabe wird erfindungsgemäss durch ein Instrumentarium mit den kennzeichnenden Merkmalen des Patentanspruches 1 gelöst.

Das erfindungsgemäss ausgebildete Instrumentarium besteht aus einem die Extremität umfassenden Halterungsrahmen, der am Skelett des Patienten in der Umgebung der späteren Knochenschnittflächen festgelegt wird und der Referenz für den winkelgebenden Teil ist. Zur Fixierung des Halterungsrahmens wird die spätere Verankerung des zur Osteosynthese verwendeten Implantates und ein oder mehrere in den Knochen zu bohrende Stifte verwendet. Der Halterungsrahmen bildet so die Basis für ein Koordinatensystem für die Lage und den Korrekturwinkel der Korrektur-Osteotomie. Auf dem winkelgebenden Teil geführt ist die Sägeführung und ein mit dieser verbundenes Zeigersystem, welches die Projektion der mechanischen Achsenverhältnisse darstellt. Mittels Führungs- und Klemm-Mechanismen können diese Zeiger so über dem Patienten positioniert werden, dass sie die Projektion der mechanischen Achse darstellen. Änderungen der mechanischen Achse, dargestellt durch Schwenkung der Zeiger, werden somit direkt auf die Sägeführung übertragen. Die Drehachse des winkelgebenden Teils entspricht dem Kreuzungspunkt der entlang der Sägeführung durchgeführten Knochenschnitte. Mittels der Führungs- und Klemmvorrichtung ist die Drehachse frei wählbar und wird direkt am Skelett angezeigt. Somit sind alle die Operation bestimmenden Grössen direkt am Skelett einstellbar und überprüfbar und damit unabhängig von der fehlerbehafteten Planung anhand von Röntgenbildern.

Beschrieben und in den Zeichnung dargestellt ist im folgenden beispielhaft die Ausführung des Instrumentes für eine valgisierende Tibia-Korrektur-Osteotomie.

Es zeigen:

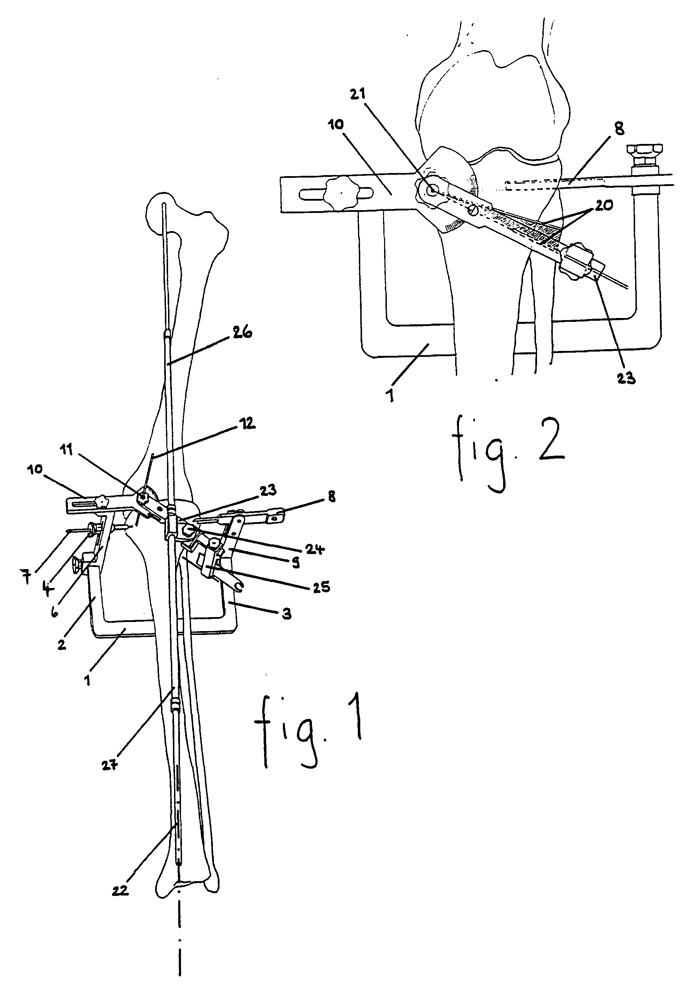

Fig. 1: ein erfindungsgemäss ausgebildetes Instrumentarium für Tibia-Korrektur-Osteotomie, positioniert an der unteren Extremität mit Skelett-Referenzen; und
Fig. 2: keilförmig angelegte Sägeschnitte der Korrektur-Osteotomie mit vorherbestimmtem Kreuzungspunkt der Schnittflächen.

Der Halterungsrahmen 1 wird beidseitig unterhalb des Knies am Skelett festgelegt. An den Schenkeln 2 und 3 des Halterungsrahmens ist die Stiftführung 6 bzw. die Halterung 9 befestigt, wobei mindestens eines dieser beiden Teile um eine Achse schwenkbar oder in einer Ebene verschiebbar angebracht ist. Im ausgeführten Beispiel ist die Klemmung 9 um die Achse des Schenkels 3 des Halterahmens 1 schwenk- und klemmbar befestigt. Ungefähr 6 bis 10 mm unterhalb der Gelenkebene wird parallel zu dieser von medial ein in der Stiftführung 6 geführter Stift 7 in den Knochen inseriert, wobei die Stiftposition entlang der Stiftführung und senkrecht zu dieser mittels einer Klemmführung 4 einstellbar ist. Lateral bildet ein in der Klemmung 9 gehaltener, in den Knochen eingeschlagener Knochenmeissel 8 die Befestigung. Für die Anwendung des Instrumentes an der linken, bzw. rechten Extremität ist die Klemmung des Knochenmeissels 8 und die Schiene für Stiftführung 6 um 180° gewendet zu montieren. Die beiden Fixierungselemente Stift 7 und Knochenmeissel 8 sind somit höhenverstellbar, wodurch der Halterungsrahmen relativ zum Skelett positionierbar ist. Der Knochenmeissel 8 kann zusätzlich zur Fixation des Instrumentes zur Vorbereitung des knöchernen Lagers für das spätere Osteosynthese-Implantat, z.B. eine gebogene Halbrohrplatte dienen. Auf der Stiftführung 6 ist verschiebbar der winkelgebende Teil 10 befestigt, wobei der Drehpunkt 11 des winkelgebenden Teils den Kreuzungspunkt 21 der Knochenschnitte 20 darstellt. Dieser Punkt wird durch einen absenkbaren Stift 12 direkt am Skelett angezeigt. Der generelle Winkel der keilförmigen Osteotomie zur tibialen Achse kann durch den Ausgangswinkel des Schwenkarmes 23 frei gewählt werden. Auf dem als Längsführung ausgebildeten Schwenkarm ist verschieb- und klemmbar die Sägeführung 25 und die in sich dreh- und klemmbare Zeigerhalterung 24 geführt. Ist das Instrument nach vorausgegangener kleiner medialer und ausgedehnter lateraler Inzision am Skelett befestigt, wird zunächst der Kreuzungspunkt der Sägeschnitte mittels Verschieben des winkelgebenden Teils 10 festgelegt. Nach Wahl des generellen Winkels der Osteotomie wird das Zeigerpaar bestehend aus dem hüftseitigen Zeiger 26 und dem schienbeinseitigen Zeiger 27 über der Mitte des Knies positioniert, parallel zur tibialen Achse 22 ausgerichtet und mittels der Zeigerhalterung 24 gegenüber dem Schwenkarm 23 geklemmt. Die Winkelposition des Schwenkarmes kann über die Winkelskala abgelesen und mittels der Klemmschraube an der Drehachse festgelegt werden. Zur Ausführung des ersten Sägeschnittes wird die Sägeführung 25 gegen den Knochen geschoben, am Schwenkarm geklemmt und mittels eines Stiftes, der durch eine entsprechende Bohrung in der Sägeführung im Knochen positioniert wird, in dieser Position gesichert.

Nach vorausgegangener Osteotomie des Wadenbeins wird der erste Sägeschnitt der keilförmigen Osteotomie angelegt. Die Stiftsicherung der Sägeführung wird gelöst und die Korrektur der Beinachse wird über den zum Hüftgelenk weisenden Zeiger eingestellt. Als Referenz dient dabei die bei der Operation leicht identifizierbare Spina iliaca anterior - superior, die 2 bis 3 cm lateral des Hüftgelenkzentrums liegt. Zur Erzielung der gewünschten Überkorrektur wird der Hüftzeiger 26 auf einen Punkt 4,5 bis 7 cm medial der Spina ausgerichtet. Diese Ausrichtmethode mit weit auseinander liegenden Referenzpunkten bietet den Vorteil geringer Fehler. Die leicht einhaltbare Messtoleranz bei der Ausrichtung der Zeiger bezüglich der Spina von einem Zentimeter ergibt einen Winkelfehler von ca. 1 bis 1,5°. Die für ein optimales Ergebnis der Operation notwendige Genauigkeit liegt bei 2 bis 3 Grad, also weit höher als der beim erfindungsgemäss ausgebildeten Instrument zu erwartende maximale Fehler.

Bei der Ausrichtung des Hüftzeigers 26 wird automatisch der Schwenkarm 23 des Instrumentes und damit die Sägeführung 25 ebenfalls um den Korrekturwinkel gedreht. Der gewählte Korrekturwinkel kann zur Kontrolle direkt auf einer Skala am winkelgebenden Teil 10 abgelesen werden. Durch die Zeiger, welche die mechanischen Achsen des Beins darstellen, kann die Korrektur direkt auf die Achsen bezogen vorgenommen werden. Der Korrekturwinkel ergibt sich dabei und kann abgelesen werden, wobei die Kenntnis dieses Winkels zur Durchführung der Operation nicht notwendig ist. Nach erfolgter Wahl der Korrektur wird die Sägeführung in der neuen Position wieder mittels des in der Bohrung der Sägeführung geführten Stiftes festgelegt und der zweite Sägeschnitt wird angelegt. Nach Entnahme des Knochenkeils wird das Instrument vom Skelett abgenommen und die Osteotomieflächen werden gegeneinander geschlossen. Das angepasste Osteosynthese-Implantat, eine gebogene Halbrohrplatte wird in dem mittels des Knochenmeissels (8) vorbereiteten Lager verankert und gegen die Tibia verschraubt. Alternativ können auch andere Implantate, z.B. Knochenklammern, verwendet werden.

## Patentansprüche

1. Instrumentarium zur Durchführung einer Umstellungsosteotomie der unteren Extremität mit einem mittels mindestens zwei Fixierungselementen am Skelett befestigbaren, offenen Halterungsrahme (1), an dem eine bezüglich dem fixierten Halterungsrahmen (1) winkelverstellbare Sägeführung (25) angeordnet ist, dadurch gekennzeichnet, dass am Halterungsrahmen (1) zwei Halteteile (6, 9) zur Führung der Fixierungselemente (7, 8) angeordnet sind, wobei an einem Halteteil (6) eine winkelgebende Skalaplatte (10) positionierbar angeklemmt ist, die von einem die Kreuzungslinie (21) der durchzuführenden Knochenschnitte (20) anzeigenden Drehachsstift (12) durchsetzt ist, und dass ein um den Drehachsstift (12) schwenkbarer Führungsarm (23) einerseits die Sägeführung (25) und andererseits winkelfest dazu zwei zueinander fluchtende, die Projektion der mechanischen Belastungsachsen der Extremität des Patienten anzeigenden Zeiger (26, 27) trägt, wobei eine Verstellung dieser Zeiger direkt eine entsprechende Verstellung der Sägeführung bewirkt.

2. Instrumentarium nach Anspruch 1, dadurch gekennzeichnet, dass der Halterungsrahmen (1) die Form eines Bügels mit zwei parallelen Schenkeln (2, 3) aufweist, an denen je eines der beiden Halteteile (6, 9) befestigt ist.

3. Instrumentarium nach Anspruch 1 oder 2, dadurch gekennzeichnet, dass als Fixierungselemente ein Stift (7) vorgesehen ist, der relativ zum Halterungsrahmen (1) in einem als Stiftführung gestalteten Halteteil (6) mittels der Klemmführung (4) höhenverstellbar gehalten ist.

4. Instrumentarium nach Anspruch 1 oder 2, dadurch gekennzeichnet, dass als Fixierungselement ein Knochenmeissel (8) vorgesehen ist, der schwenk- und klemmbar in einem Halteteil (9) gehalten ist.

5. Instrumentarium nach einem der Ansprüche 2 bis 4, dadurch gekennzeichnet, dass mindestens eine der beiden Halterungen (6, 9) auf dem entsprechenden Schenkel (2, 3) des Halterungsrahmens verschieb- oder schwenkbar gehalten ist.

6. Instrumentarium nach einem der Ansprüche 1 bis 5, dadurch gekennzeichnet, dass das Zeigerpaar (26, 27) mittels eines Befestigungselementes (24) winkelfest mit der Sägeführung verbindbar ist.

7. Instrumentarium nach einem der Ansprüche 1 - 6, dadurch gekennzeichnet, dass die Zeiger (26, 27) verlängerbar sind.

8. Instrumentarium nach Anspruch 7, dadurch gekennzeichnet, dass die verlängerbaren Zeiger (26, 27) teleskopisch ausgebildet sind.

## Claims

1. Instruments for carrying out of an adjustment osteotomy of the lower extremity with an open securing frame (1) attachable to the skeleton by means of at least two fixation elements, where a saw guide which is angle-adjustable in relation to the fixed securing frame (1) is arranged, characterized in that at the securing frame (1) two securing components (6;9) to the guidance of the fixation elements (7;8) are arranged, whereby at one securing component (6) an angle-determining scale plate is positionably clamped which is interspersed with a rotation axis pin (12) that indicates the crossing line (21) of the bone cuts (20) to be carried out and that an angle-determining component (23) which is pivotable around the rotation axis pin (12) carries on the one hand the saw guide (25) and on the other hand two pointers (26;27) which are in alignment to each other and indicating the projection of the mechanical loading axis of the extremity of the patient whereby an adjustment of these pointers causes directly a corresponding adjustment of the saw guide.

2. Instruments according to claim 1, characterized in that the securing frame (1) shows the shape of a bow with two parallel legs (2;3) at each having one of the two securing components (6;9) attached.

3. Instruments according to claim 1 or 2, characterized in that as a fixation element a pin (7) is provided in a securing component (6) which is shaped as a pin guidance by means of the clamping guidance (4) whereby said pin (7) is height adjustable relative to the securing frame (1).

4. Instruments according to claim 1 or 2, characterized in that as a fixation element a bone chisel (8) is provided that must be pivotable and clampable in a securing component (9).

5. Instruments according to one of the claims 2 to 4, characterized in that at least one of the securing components (6;9) is moveable or pivotable on the corresponding leg (2;3) of the securing frame.

6. Instruments according to one of the claims 1 to 5, characterized in that the pair of pointers (26;27) is angle-determinedly connectable with the saw guidance by means of a fastening element (24).

7. Instruments according to one of the claims 1 to 6, characterized in that the pointers (26;27) are lengthenable.

8. Instruments according to claim 7, characterized in that the lengthenable pointers (26;27) are formed telescopically.

## Revendications

1. Instrumentation en vue de la réalisation d'une ostéotomie de correction de l'extrémité inférieure, présentant un cadre de maintien (1) ouvert, apte à être fixé au squelette au moyen d'au moins deux éléments de fixation, sur lequel un guide - scie (25) apte à être déplacé angulairement par rapport au cadre de maintien (1) est disposé fixement, caractérisée en ce que deux pièces de maintien (6, 9) pour le guidage des éléments de fixation (7, 8) sont disposées sur le cadre de maintien (1), une plaque à échelle (10) d'indication de l'angle apte à être serrée sur une pièce de maintien (6) étant traversée par une tige d'axe de rotation (12) indiquant la ligne de croisement (21) de la découpe (20) à réaliser dans l'os, et en ce qu'un bras de guidage (23) apte à pivoter autour de la tige de l'axe de rotation (12) porte d'une part le guide - scie (25) et d'autre part, à angle fixe par rapport à ce dernier, deux indicateurs (26, 27) alignés l'un sur l'autre et indiquant la projection de l'axe mécanique de charge de l'extrémité du patient, un déplacement de ces indicateurs ayant pour effet direct un déplacement correspondant du guide - scie.

2. Instrumentation selon la revendication 1, caractérisée en ce que le cadre de maintien (1) présente la forme d'un étrier présentant deux branches parallèles (2, 3) à chacune desquelles est fixée l'une des deux pièces de maintien (6, 9).

3. Instrumentation selon la revendication 1 ou 2, caractérisée en ce que comme élément de fixation est prévue une tige (7) qui est maintenue au moyen du guide de serrage (4) dans une pièce de maintien (6) configurée comme guide - tige, de manière ajustable en hauteur par rapport au cadre de maintien (1).

4. Instrumentation selon la revendication 1 ou 2, caractérisée en ce que comme élément de fixation est prévu un ciseau à os (8) qui est maintenu de manière à pouvoir pivoter et à être serré dans une pièce de maintien (9).

5. Instrumentation selon l'une des revendications 2 à 4, caractérisée en ce qu'au moins l'un des deux supports (6, 9) est maintenu de manière à pouvoir coulisser ou pivoter sur la branche (2, 3) correspondante du cadre de maintien.

6. Instrumentation selon l'une des revendications 1 à 5, caractérisée en ce que la paire d'indicateurs (26, 27) peut être reliée à angle fixe au guide scie au moyen d'un élément de fixation (24).

7. Instrumentation selon l'une des revendications 1 à 6, caractérisée en ce que les indicateurs (26, 27) peuvent être prolongés.

8. Instrumentation selon la revendication 7, caractérisée en ce que les indicateurs (26, 27) aptes à être prolongés sont télescopiques.
